# EUROPÄISCHE PATENTSCHRIFT

(11) **EP 3 497 391 B1**
(45) Veröffentlichungstag und Bekanntmachung des Hinweises auf die Patenterteilung: **03.06.2020**
(21) Anmeldenummer: 17749737.7
(22) Anmeldetag: 14.08.2017
(51) Int. Cl.: C07C 7/04, C07C 7/09, C07C 7/11, C07C 11/04, B01D 3/14, B01D 53/00, B01D 53/14, F25J 3/02

(54) **VERFAHREN ZUR GEWINNUNG EINES ÜBERWIEGEND KOHLENWASSERSTOFFE MIT ZWEI KOHLENSTOFFATOMEN ENTHALTENDEN TRENNPRODUKTS**
METHOD FOR OBTAINING A SEPARATION PRODUCT CONTAINING MOSTLY HYDROCARBONS WITH 2 CARBON ATOMS
PROCEDE DE PRODUCTION D'UN PRODUIT DE SEPARATION CONTENANT PRINCIPALEMENT DES HYDROCARBURES A DEUX ATOMES DE CARBONE

(30) Priorität: 12.08.2016 EP 16183935
(43) Veröffentlichungstag der Anmeldung: 19.06.2019
(73) Patentinhaber: Linde AG, 80331 München (DE)
(72) Erfinder: PHAM DUC, Tuat, 82377 Penzberg (DE); SPINDELNDREHER, Anne, 82377 Penzberg (DE); KURZ, Benedikt, 80686 München (DE)
(74) Vertreter: Gellner, Bernd
(86) Internationale Anmeldenummer: PCT/EP2017/070568
(87) Internationale Veröffentlichungsnummer: WO 2018/029380

(56) Entgegenhaltungen:
- EP-A1- 3 029 017
- EP-A1- 3 029 402
- WO-A1-2015/104153
- WO-A2-2007/045364
- DE-A1-102010 014 155

## Beschreibung

Die Erfindung betrifft ein Verfahren und eine Anlage zur Gewinnung eines überwiegend Kohlenwasserstoffe mit zwei Kohlenstoffatomen enthaltenden Trennprodukts gemäß den Oberbegriffen der unabhängigen Patentansprüche.

### Stand der Technik

Verfahren und Vorrichtungen zum Dampfspalten (engl. Steam Cracking) und zur Aufbereitung der hierbei gewonnenen Gasgemische sind aus dem Stand der Technik in unterschiedlichen Ausgestaltungen bekannt. Zu weiteren Details sei auf einschlägige Fachliteratur, beispielsweise den Artikel "Ethylene" in Ullmann's Encyclopedia of Industrial Chemistry, Onlineausgabe, 2009, DOI 10.2002/14356007.a10_045.pub3 oder Falqi, F.: "The Miracle of Petrochemicals. Olefins Industry: An In-Depth Look at Steam-Crackers", Universal-Publishers 2009, ISBN 1-59942-915-2, verwiesen.

Dampfspaltverfahren werden im kommerziellen Maßstab in Rohrreaktoren durchgeführt, die grundsätzlich mit einer Vielzahl von Kohlenwasserstoffen und Kohlenwasserstoffgemischen von Ethan bis Gasöl bis zu einem Siedepunkt von typischerweise 600 °C (sogenannter Einsatz) beschic kt werden können. Der Einsatz wird beim Dampfspalten in dem oder den Rohrreaktoren zumindest teilweise umgesetzt, wodurch ein sogenanntes Rohgas erhalten wird. Das Rohgas kann einer Reihe von Nachbehandlungsschritten unterworfen werden. Derartige Nachbehandlungsschritte umfassen typischerweise eine Aufbereitung des Rohgases durch Kühlen und Trocknen, wodurch ein sogenanntes Spaltgas erhalten wird. Bisweilen wird auch das Rohgas als Spaltgas bezeichnet und umgekehrt.

Bei dem Spaltgas handelt es sich um ein Kohlenwasserstoffgemisch mit Kohlenwasserstoffen unterschiedlicher Kettenlänge und Struktur. Um aus dem Spaltgas die erwünschten Produkte zu gewinnen, muss dieses daher aufgetrennt werden. Aus dem Stand der Technik sind hierzu unterschiedliche Verfahren bekannt und beispielsweise in dem erwähnten Artikel "Ethylene" in Ullmann's Encyclopedia of Industrial Chemistry ausführlich beschrieben.

In einem ersten Trennschritt werden typischerweise benzin- und ölartige Komponenten, falls in dem Spaltgas vorhanden, abgetrennt. Hierzu kommen typischerweise Öl- und Wasserwäschen zum Einsatz. Nachfolgend kann beispielsweise zunächst ein Gasgemisch, das überwiegend Methan, Wasserstoff und Kohlenwasserstoffe mit zwei Kohlenstoffatomen enthält, aus dem Spaltgas abgetrennt werden. Man spricht dann auch von einem "Deethanizer First"- oder "Frontend Deethanizer"-Verfahren. Aus der Fachliteratur sind jedoch auch beispielsweise sogenannte "Demethanizer First"- und "Depropanizer First"-Verfahren bekannt.

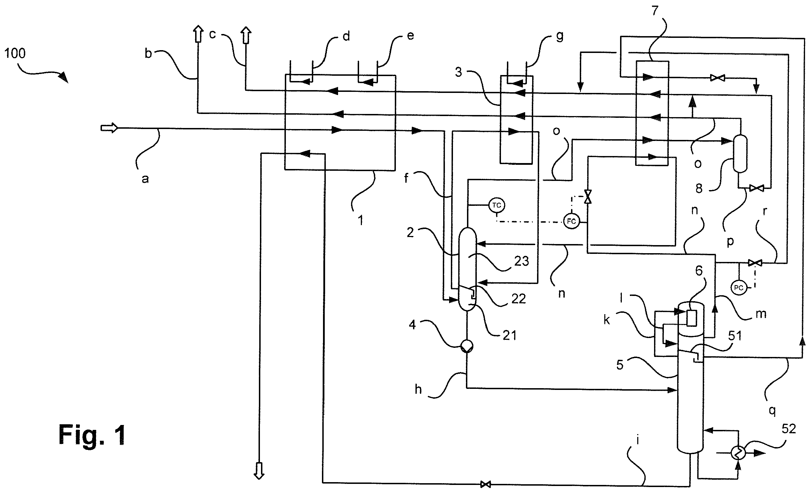

Zur Abtrennung der Kohlenwasserstoffe mit zwei Kohlenstoffatomen aus Gasgemischen, die überwiegend Methan, Wasserstoff und Kohlenwasserstoffe mit zwei Kohlenstoffatomen enthalten, und die aus dem Spaltgas eines Dampfspaltverfahrens gebildet wurden, können Trennverfahren eingesetzt werden, wie sie in der EP 3 029 017 A1 unter Bezugnahme auf die Figuren 1 und 2 erläutert sind. Auch die WO 2015/104153 A1 zeigt ein entsprechendes Verfahren. Solche herkömmlichen Verfahren eignen sich jedoch vor allem für solche Gasgemische, die aus einem Spaltgas gebildet werden, das sich beim Dampfspalten von Naphtha und/oder schwereren Kohlenwasserstoffen als Einsatz bildet, da nur in diesem Fall ausreichend (mindestens 30 Molprozent) Methan enthalten ist. Entsprechend hohe Methanmengen werden herkömmlicherweise benötigt, um einen Rücklauf auf den Demethanizer und den C2-Absorber (siehe hierzu auch Erläuterungen zu Figur 1) bereitzustellen.

Weitere Verfahren zur Bearbeitung von Spaltgasen sind aus der EP 3 029 402 A1, der WO 2007/045364 A2, der DE 10 2010 014 155 A1 und der WO 2017/001514 A1 bekannt.

Werden beim Dampfspalten jedoch gemischte Einsätze verwendet, d.h. wird neben Naphtha auch gasförmiger Einsatz wie Ethan gespalten, enthält das Spaltgas und damit auch das daraus abgetrennte, überwiegend Methan, Wasserstoff und Kohlenwasserstoffe mit zwei Kohlenstoffatomen enthaltende Gasgemisch, vergleichsweise wenig Methan. Somit können der Demethanizer und der C2-Absorber nicht ohne weiteres betrieben werden. Für diesen Fall muss in herkömmlichen Anlagen ein Teil des Methans nach der Trennung von Wasserstoff und den Kohlenwasserstoffen mit zwei Kohlenstoffatomen zurückgeführt werden. Durch eine entsprechende Rückführung lässt sich Methan im Kreislauf anreichern. Je nachdem, wie groß das Verhältnis von Ethan zu Naphtha im Einsatz ist, kann die Rückführung bis zu 15 Gewichtsprozent des Spaltgases betragen. Dies führt dazu, dass alle trenntechnischen Apparate des Roh- bzw. Spaltgasweges entsprechend größer ausgelegt werden müssen und die Anlage entsprechend mehr Verdichterleistung verbraucht. Beides ist nachteilig.

Eine in der EP 3 029 017 A1 vorgeschlagene Lösung besteht in der externen Bereitstellung von Methan. Dieses muss jedoch hierbei stets in ausreichender Menge und in geeignetem Lieferzustand zur Verfügung stehen.

Damit stellt sich vorliegend die Aufgabe, die Gewinnung überwiegend Kohlenwasserstoffe mit zwei Kohlenstoffatomen enthaltender Trennprodukte aus überwiegend Methan, Wasserstoff und Kohlenwasserstoffe mit zwei Kohlenstoffatomen enthaltenden Trenneinsätzen, wie sie aus Spaltgasen von Dampfspaltverfahren gebildet werden, zu verbessern.

### Offenbarung der Erfindung

Diese Aufgabe wird durch ein Verfahren und eine Anlage zur Gewinnung eines überwiegend Kohlenwasserstoffe mit zwei Kohlenstoffatomen enthaltenden Trenneinsatzes mit den Merkmalen der unabhängigen Patentansprüche gelöst. Ausgestaltungen sind jeweils Gegenstand der abhängigen Patentansprüche sowie der nachfolgenden Beschreibung.

Vor der Erläuterung der Merkmale und Vorteile der vorliegenden Erfindung werden deren Grundlagen und die verwendeten Begriffe erläutert.

Flüssige und gasförmige Stoffgemische, Fraktionen und dergleichen können im hier verwendeten Sprachgebrauch reich oder arm an einer oder mehreren Komponenten sein, wobei hier "reich" für einen Gehalt von wenigstens 90%, 95%, 99%, 99,5%, 99,9% oder 99,99% und "arm" für einen Gehalt von höchstens 10%, 5%, 1%, 0,1% oder 0,01% auf molarer, Gewichts- oder Volumenbasis stehen kann. Ist hier davon die Rede, dass Stoffgemische, Fraktionen und dergleichen "überwiegend" aus einer oder mehreren Komponenten bestehen, sind sie "reich" hieran im soeben erläuterten Sinn. Ein Stoffgemisch, das beispielsweise "reich" an Methan und Wasserstoff ist, enthält also zu wenigstens 90%, 95%, 99%, 99,5%, 99,9% oder 99,99% Methan und Wasserstoff und nur im ggf. verbleibenden Anteil sonstige Komponenten. Ist vorliegend beispielsweise von "Methan" oder "Wasserstoff' die Rede, sei darunter ein Fluid verstanden, das reich an der entsprechenden Komponente ist, jedoch nicht ausschließlich aus dieser bestehen muss.

Flüssige und gasförmige Stoffgemische können im hier verwendeten Sprachgebrauch ferner angereichert oder abgereichert an einer oder mehreren Komponenten sein, wobei sich diese Begriffe auf einen entsprechenden Gehalt in einem Ausgangsgemisch beziehen, aus dem das Stoffgemisch erhalten wurde. Das Stoffgemisch ist dabei im hier verwendeten Sprachgebrauch "angereichert", wenn es zumindest den 1,1-fachen, 1,5-fachen, 2-fachen, 5-fachen, 10-fachen, 100-fachen oder 1.000-fachen Gehalt, hingegen "abgereichert", wenn es höchstens den 0,9-fachen, 0,5-fachen, 0,1-fachen, 0,01-fachen oder 0,001-fachen Gehalt einer entsprechenden Komponente, bezogen auf das Ausgangsgemisch, enthält.

Im Rahmen der vorliegenden Erfindung kommen Rektifikations- und Absorptionskolonnen zum Einsatz. Zur Auslegung und Ausgestaltung entsprechender Apparate sei auf einschlägige Lehrbücher verwiesen (siehe beispielsweise K. Sattler: Thermische Trennverfahren. Grundlagen, Auslegung, Apparate. Weinheim: Wiley-VCH, 3. Auflage 2001). Einer Rektifikations- und einer Absorptionskolonne ist typischerweise immer zumindest eine flüssige Fraktion ("Sumpfflüssigkeit") und eine gasförmige Fraktion ("Kopfgas") aus einem unteren Bereich ("Sumpf) bzw. aus einem oberen Bereich ("Kopf") entnehmbar.

Bei einer "Rektifikationskolonne" handelt es sich im hier verwendeten Sprachgebrauch um eine Trennsäule, die dafür eingerichtet ist, ein gasförmig oder flüssig oder in Form eines Zweiphasengemischs mit flüssigen und gasförmigen Anteilen, ggf. auch im überkritischen Zustand, bereitgestelltes Stoffgemisch zumindest teilweise aufzutrennen, also aus dem Stoffgemisch jeweils Reinstoffe oder Stoffgemische zu erzeugen, die gegenüber dem eingesetzten Stoffgemisch bezüglich zumindest einer Komponente angereichert bzw. abgereichert oder reich bzw. arm im oben erläuterten Sinne sind. Typischerweise sind Rektifikationskolonnen als zylindrische Metallbehälter ausgebildet, die mit Einbauten, beispielsweise Siebböden oder geordneten oder ungeordneten Packungen, ausgerüstet sind. Eine Rektifikationskolonne zeichnet sich unter anderem dadurch aus, dass das Sumpfprodukt mittels eines Sumpfverdampfers erwärmt wird, so dass kontinuierlich ein Teil verdampft und in der Rektifikationskolonne gasförmig aufsteigt. Eine Rektifikationskolonne ist ferner typischerweise mit einem sogenannten Kopfkondensator versehen, in den zumindest ein Teil des Kopfgases zu einem Kondensat verflüssigt und als flüssiger Rücklauf am Kopf der Rektifikationskolonne aufgegeben wird. Ein Teil des Kopfgases kann aber auch anderweitig, beispielsweise als Produkt, verwendet werden.

Im Gegensatz zu einer Rektifikationskolonne verfügt eine "Absorptionskolonne" typischerweise nicht über einen Sumpfverdampfer. Auch Absorptionskolonnen sind aus dem Bereich der Trenntechnik allgemein bekannt. Absorptionskolonnen werden zur Absorption im Phasengegenstrom verwendet und daher auch als Gegenstromkolonnen bezeichnet. Bei der Absorption im Gegenstrom strömt die abgebende Gasphase aufwärts durch eine Absorptionskolonne. Die aufnehmende Lösungsphase fließt, von oben aufgegeben und unten abgezogen, der Gasphase entgegen. Die Gasphase wird mit der Lösungsphase "gewaschen". In einer entsprechenden Absorptionskolonne sind ebenfalls typischerweise Einbauten vorgesehen, die für einen stufenweisen (Böden, Sprühzonen, rotierende Teller usw.) oder stetigen (regellose Schüttungen von Füllkörpern, Packungen usw.) Phasenkontakt sorgen. In einen oberen Bereich wird ein flüssiger Strom, hier als "Absorptionsflüssigkeit" bezeichnet, eingespeist, mittels derer Komponenten aus einem gasförmigen Strom, der in einen unteren Bereich der Absorptionskolonne eingespeist werden, ausgewaschen werden.

Die vorliegende Anmeldung verwendet zur Charakterisierung von Drücken und Temperaturen die Begriffe "Druckniveau" und "Temperaturniveau", wodurch zum Ausdruck gebracht werden soll, dass entsprechende Drücke und Temperaturen in einer entsprechenden Anlage nicht in Form exakter Druck- bzw. Temperaturwerte verwendet werden müssen, um das erfinderische Konzept zu verwirklichen. Jedoch bewegen sich derartige Drücke und Temperaturen typischerweise in bestimmten Bereichen, die beispielsweise ± 1%, 5%, 10%, 20% oder sogar 50% um einen Mittelwert liegen. Entsprechende Druckniveaus und Temperaturniveaus können dabei in disjunkten Bereichen liegen oder in Bereichen, die einander überlappen. Insbesondere schließen beispielsweise Druckniveaus unvermeidliche oder zu erwartende Druckverluste, beispielsweise aufgrund von Abkühlungseffekten, ein. Entsprechendes gilt für Temperaturniveaus. Bei den hier in bar angegebenen Druckniveaus handelt es sich um Absolutdrücke.

Im Rahmen der vorliegenden Erfindung kommt unter anderem Ethylen auf unterschiedlichen Druckniveaus als Kältemittel zum Einsatz. Eine entsprechende Verwendung von Ethylen ist aus dem Stand der Technik weitgehend bekannt. In aus dem Stand der Technik bekannten Kältemittelkreisläufen wird Ethylen auf drei Druckniveaus, einem Druckniveau von ca. 7 bis 9 bar ("Hochdruckethylen"), auf einem Druckniveau von ca. 3 bis 4 bar ("Mitteldruckethylen") und auf einem Druckniveau von ca. 1,15 bis 1,4 bar ("Niederdruckethylen") bereitgestellt. Mit Hochdruckethylen alleine kann dabei ein Temperaturniveau von ca. -62 bis -55 °C, mit Mitteldruckethylen alleine ein Temperaturniveau von ca. -83 bis bis -76 °C und mit Niederdruckethylen alleine ein Temperaturniveau von ca. -102 bis -98 °C erreicht werden.

### Vorteile der Erfindung

Grundsätzlich beruht das im Rahmen der vorliegenden Erfindung vorgeschlagene Verfahren auf den aus dem Stand der Technik bekannten Konzepten zur Gewinnung überwiegend Kohlenwasserstoffe mit zwei Kohlenstoffatomen enthaltender Trennprodukte aus überwiegend Methan, Wasserstoff und Kohlenwasserstoffe mit zwei Kohlenstoffatomen enthaltenden Trenneinsätzen, die eine (Teil-)Kondensation, eine Gegenstromabsorption und eine Rektifikation umfassen.

Die vorliegende Erfindung beruht nun aber auf der Erkenntnis, dass es besonders vorteilhaft ist, für den eingangs erwähnten Zweck, nämlich die Bearbeitung entsprechender Gasgemische, die vergleichsweise geringe Mengen an Methan enthalten, sowohl eine spezifische, modifizierte Einsatzabkühlung als auch ein bestimmtes (nämlich gegenüber der Abkühlung erhöhtes) Druckniveau bei der Tieftemperaturrektifikation, auf dem die in einem entsprechenden Verfahren eingesetzte Rektifikationskolonne ("Demethanizer") betrieben wird, einzusetzen. Gemäß einer Ausgestaltung der vorliegenden Erfindung wird für die Gegenstromabsorption ferner eine modifizierte Absorptionskolonne eingesetzt.

Im Rahmen der vorliegenden Erfindung beträgt der Methangehalt des Trenneinsatzes bis zu 30% (insbesondere auf molarer Basis) und der Trenneinsatz wird in gasförmigem Zustand bereitgestellt. Der Methangehalt kann beispielsweise 20 bis 25 oder 25 bis 30 Molprozent betragen. Der Trenneinsatz wird im Rahmen der Erfindung auf einem ersten Druckniveau durch Abkühlen von einem ersten Temperaturniveau auf ein zweites Temperaturniveau unter Gewinnung genau einer ersten Flüssigfraktion und genau einer ersten Gasfraktion einmalig teilkondensiert. Bevorzugte Werte für die Druck- und Temperaturniveaus sind unten angegeben.

Wird ein entsprechender Trenneinsatz, wie in der vorliegenden Erfindung, nicht stufenweise sowie unter mehrfacher Abscheidung von Kondensaten, wie beispielsweise in der EP 3 029 017 A1 unter Bezugnahme auf die Figuren 1 und 2 erläutert, sondern einstufig unter nur einmaliger Abscheidung eines Kondensats abgekühlt, geht eine größere Menge an Methan in die abgeschiedene flüssige Fraktion, hier also die "erste Flüssigfraktion", über als bei Abkühlungen mit zwischen Abscheidung der Kondensate. Dies ist darauf zurückzuführen, dass der Trenneinsatz bei höherem Partialdruck des Methans bis auf das tiefste Temperaturniveau abgekühlt wird. Dies will man in herkömmlichen Verfahren, in denen höhere Methanmengen zur Verfügung stehen, gerade vermeiden, weil Methan, das bereits durch die Kondensation abgeschieden wird, in der späteren Trennung nicht mehr abgetrennt werden muss.

Die erste Flüssigfraktion ist gegenüber dem Trenneinsatz an Kohlenwasserstoffen mit zwei Kohlenstoffatomen angereichert. Dennoch ist in der ersten Flüssigfraktion aus den erläuterten Gründen mehr Methan enthalten als in den entsprechenden Flüssigfraktionen, die im Stand der Technik durch eine mehrstufige Teilkondensation abgeschieden werden. Die erste Gasfraktion enthält hingegen nahezu den gesamten Wasserstoff aus dem Trenneinsatz und ist gegenüber diesem an Kohlenwasserstoffen mit zwei Kohlenstoffatomen abgereichert, an Methan hingegen angereichert. Die weitere Behandlung der ersten Gasfraktion dient anschließend im Wesentlichen der Rückgewinnung der Kohlenwasserstoffe mit zwei Kohlenstoffatomen.

Im Rahmen der vorliegenden Erfindung wird dazu zumindest ein Teil der ersten Gasfraktion auf dem ersten Druckniveau durch weiteres Abkühlen auf ein drittes Temperaturniveau unter Erhalt genau einer zweiten Flüssigfraktion und genau einer zweiten Gasfraktion einmalig teilkondensiert. Auf diese Weise scheidet sich ein weiterer Anteil der Kohlenwasserstoffe mit zwei Kohlenstoffatomen aus der ersten Gasfraktion in der zweiten Flüssigfraktion ab. In der zweiten Gasfraktion sind jedoch noch weiterhin Kohlenwasserstoffe mit zwei Kohlenstoffatomen enthalten.

Daher wird nun zumindest ein Teil der zweiten Gasfraktion auf dem ersten Druckniveau unter Erhalt genau einer dritten Flüssigfraktion und genau einer dritten Gasfraktion einer Gegenstromabsorption im Gegenstrom zu einer überwiegend Methan enthaltenden Absorptionsflüssigkeit unterworfen. Mittels der überwiegend Methan enthaltenden Absorptionsflüssigkeit werden die Kohlenwasserstoffe mit zwei Kohlenstoffatomen weitgehend aus der zweiten Gasfraktion ausgewaschen. Es kommt also ein sogenannter C2-Absorber zum Einsatz.

Die erste, die zweite und die dritte Flüssigfraktion, die Methan und Kohlenwasserstoffe mit zwei Kohlenstoffatomen enthalten, werden nun einer Tieftemperaturrektifikation unterworfen, die auf einem erhöhten Druckniveau erfolgt. Dazu werden die erste, die zweite und die dritte Flüssigfraktion zumindest teilweise vereinigt und zumindest teilweise auf einem zweiten Druckniveau oberhalb des ersten Druckniveaus unter Erhalt einer Sumpfflüssigkeit und eines Kopfgases der Tieftemperaturrektifikation unterworfen. Das erhöhte zweite Druckniveau wird im Rahmen der vorliegenden Erfindung deshalb eingesetzt, damit sichergestellt werden kann, dass Methan am Kopf der verwendeten Rektifikationskolonne (des Demethanizers) mit Niederdruckethylen kondensiert werden kann und keine kälteren Kältemittel wie expandierendes Methan eingesetzt werden müssen. Auch auf diese Weise kann die geringere Methanmenge in dem Trenneinsatz adressiert werden.

Im Rahmen der vorliegenden Erfindung wird dabei zumindest ein Teil des Kopfgases auf dem zweiten Druckniveau durch weiteres Abkühlen auf ein viertes Temperaturniveau unter Erhalt einer vierten Flüssigfraktion und einer vierten Gasfraktion, insbesondere genau einer vierten Flüssigfraktion und genau einer vierten Gasfraktion, insbesondere einmalig, teilkondensiert. Dies erfolgt typischerweise in einem Kopfkondensator der Rektifikationskolonne. Das vierte Temperaturniveau wird dabei vorteilhafterweise mit Niederdruckethylen erzielt, wie erläutert.

Im Rahmen der vorliegenden Erfindung wird die überwiegend Methan enthaltende Absorptionsflüssigkeit durch weiteres Abkühlen zumindest eines Teils der vierten Gasfraktion auf ein fünftes Temperaturniveau gebildet, bei dem vorteilhafterweise eine nahezu vollständige Kondensation erfolgt. Die hierzu vorteilhafterweise verwendete Vorgehensweise ist unten erläutert.

Im Rahmen der vorliegenden Erfindung liegt das erste Temperaturniveau bei -20 bis - 35 °C, insbesondere bei -23 bis -32 °C und das zwei te Temperaturniveau bei -75 bis -80 °C, insbesondere bei -77 bis -79 °C. Das dritte Temperaturniveau liegt vorteilhafterweise bei -100 bis -105 °C, insbesonde re bei -100 bis -102 °C und/oder das vierte Temperaturniveau vorteilhafterweise bei -95 bis -101 °C, insbesondere bei -97 bis -99 °C. Das fünfte Temperaturniveau liegt vorteilhafterweise bei -140 bis -155 °C, insbesondere bei -148 bis -152 °C. Ferner liegt das erste Druckniveau vorteilhafterweise bei 32 bis 37 bar, insbesondere bei 35 bis 37 bar und/oder das zweite Druckniveau vorteilhafterweise bei 35 bis 40 bar, insbesondere bei 35 bis 37 bar. Die Vorteile der im Rahmen der vorliegenden Erfindung spezifisch verwendeten Druck- und Temperaturniveaus wurden bereits erläutert. Weitere Beispiele für Druck- und Temperaturniveaus sind unter Bezugnahme auf die Figur 1 erläutert.

Gemäß einer besonders bevorzugten Ausführungsform des erfindungsgemäßen Verfahrens wird für die Gegenstromabsorption eine Absorptionskolonne verwendet, die einen Sumpfbereich und einen von dem Sumpfbereich durch eine Flüssigkeitssperre getrennten Absorptionsbereich, der oberhalb des Sumpfbereichs angeordnet ist, aufweist, wobei die Flüssigkeitssperre derart ausgebildet ist, dass sie Flüssigkeit, die sich in einem unteren Bereich des Absorptionsbereichs an der Flüssigkeitssperre sammelt, in den Sumpfbereich abfließen lässt und dabei ein Aufsteigen von Gas aus dem Sumpfbereich in den Absorptionsbereich verhindert. Auf diese Weise lässt sich die Phasentrennung der ersten Gasfraktion von der ersten Flüssigfraktion, der zweiten Gasfraktion von der zweiten Flüssigfraktion und die Gegenstromabsorption zur Bildung der dritten Gasfraktion und der dritten Flüssigfraktion auf besonders vorteilhafte Weise in einem einzigen trenntechnischen Apparat vornehmen. Dieser lässt sich wirtschaftlich besonders effizient erstellen und vorteilhaft betreiben. Die Flüssigkeitssperre arbeitet dabei nach Art eines Siphons, der Flüssigkeit abfließen, aber kein Gas aufsteigen lässt. Er kann nach Art eines aus dem Bereich der Rektifikation bekannten Siphonbodens ausgebildet sein, wobei jedoch auf einen Gasdurchlass verzichtet wird.

Wird eine derartige, modifizierte Absorptionskolonne eingesetzt, kann der von dem ersten Temperaturniveau auf das zweite Temperaturniveau abgekühlte Trenneinsatz als Zweiphasengemisch in den Sumpfbereich eingespeist werden, wo sich sodann die erste Flüssigfraktion von der ersten Gasfraktion trennt. Die Flüssigfraktion muss dabei nicht in Form einer selbständigen Fraktion vorliegen, sondern kann sich bereits während ihrer Bildung mit der durch die Flüssigkeitssperre aus dem Absorptionsbereich abfließenden Flüssigkeit mischen.

Wird eine modifizierte Absorptionskolonne eingesetzt, kann ferner die auf das dritte Temperaturniveau weiter abgekühlte erste Gasfraktion oder deren Teil als Zweiphasengemisch sumpfseitig in den Absorptionsbereich eingespeist werden, so dass sich dort die zweite Flüssigfraktion von der zweiten Gasfraktion trennt. Auch hierbei muss die zweite Flüssigfraktion nicht in Form einer selbständigen Fraktion anfallen sondern sie kann sich bei ihrer Bildung mit herabrieselnder, beladener Waschflüssigkeit mischen, die, wie erwähnt, hier auch als dritte Flüssigfraktion bezeichnet wird. Mit anderen Worten kann bei Einsatz einer entsprechend modifizierten Absorptionskolonne die dritte Flüssigfraktion mit der zweiten Flüssigfraktion bei deren Bildung oberhalb der Flüssigkeitssperre vereinigt und über die Flüssigkeitssperre in den Sumpfbereich abgelassen werden, wo sie bei deren Bildung mit der ersten Flüssigfraktion vereinigt wird.

Im Rahmen der vorliegenden Erfindung werden die erste, die zweite und die dritte Flüssigfraktion oder deren vereinigte Anteile vorteilhafterweise mittels einer Sumpfpumpe druckerhöht und in eine für die Tieftemperaturrektifikation verwendete Rektifikationskolonne überführt. Dadurch, dass die bei der Abkühlung anfallenden Kondensate auf Druck gebracht werden, kann eine energieaufwendige Druckerhöhung des gesamten Trenneinsatzes vermieden werden. Zwar ist eine entsprechende Pumpe erforderlich, allerdings kann aus den erläuterten Gründen beispielsweise auf Methankältemittel oder die Entspannung von entsprechenden Strömen und eine anschließende Rückverdichtung verzichtet werden.

Im Rahmen der vorliegenden Erfindung ist es, wie erwähnt, vorteilhaft, wenn zur Teilkondensation des Kopfgases oder dessen Teils ein Kopfkondensator der Rektifikationskolonne verwendet wird, der unter Verwendung von Niederdruckethylen gekühlt wird. Durch den erhöhten Betriebsdruck in der Rektifikationskolonne ist eine Kondensation mit Niederdruckethylen möglich, so dass auf kältere Kältemittel verzichtet werden kann.

Im Rahmen der vorliegenden Erfindung wird vorteilhafterweise zumindest ein Teil der dritten Gasfraktion auf dem ersten Druckniveau durch Abkühlen auf das fünfte Temperaturniveau unter Erhalt einer fünften Flüssigfraktion und einer fünften Gasfraktion, insbesondere genau einer fünften Flüssigfraktion und genau einer fünften Gasfraktion, insbesondere einmalig, teilkondensiert. Auf diese Weise kann eine Trennung in eine methanangereicherte oder methanreiche Fraktion (die fünfte Flüssigfraktion) und eine wasserstoffangereicherte oder wasserstoffreiche Fraktion (die fünfte Gasfraktion) bewirkt werden. Die Fraktionen können weiter behandelt werden.

Besonders vorteilhaft ist es dabei, wenn zur Abkühlung der dritten und der vierten Gasfraktion oder deren Teilen zumindest ein Wärmetauscher verwendet wird, der unter Verwendung zumindest eines Teils der fünften Flüssigfraktion und der fünften Gasfraktion gekühlt wird. Auf diese Weise lassen sich zur Abkühlung der genannten Gasfraktionen jeweils Temperaturen, nämlich das fünfte Temperaturniveau, erzielen, die unter Verwendung von Niederdruckethylen nicht erzielbar wären.

Im Rahmen der vorliegenden Erfindung kommt zur Abkühlung des Trenneinsatzes vorteilhafterweise zumindest ein Wärmetauscher zum Einsatz, der unter Verwendung zumindest eines Teils der fünften Flüssigfraktion und der fünften Gasfraktion sowie mit Hoch- und Mitteldruckethylen gekühlt wird. Auf diese Weise lässt sich die Temperatur des Trenneinsatzes ausreichend absenken, ohne auf übermäßige Mengen von externem Kältemittel zurückgreifen zu müssen.

Zur Abkühlung der ersten Gasfraktion wird hingegen wenigstens ein Wärmetauscher verwendet, der, insbesondere zusammen mit zumindest einem Teil der fünften Flüssigfraktion und der fünften Gasfraktion, mit Niederdruckethylen gekühlt wird. Auf diese Weise lässt sich das dritte Temperaturniveau erreichen.

Eine Anlage zur Gewinnung eines Trennprodukts, das überwiegend Kohlenwasserstoffe mit zwei Kohlenstoffatomen enthält, unter Verwendung eines Trenneinsatzes, der überwiegend Methan, Wasserstoff und Kohlenwasserstoffe mit zwei Kohlenstoffatomen enthält, wobei der Methangehalt des Trenneinsatzes bis zu 20% beträgt und der Trenneinsatz in gasförmigem Zustand bereitgestellt wird, kann eingesetzt werden.

Diese Anlage zeichnet sich durch Mittel aus, die dazu eingerichtet sind, den Trenneinsatz auf einem ersten Druckniveau durch Abkühlen von einem ersten Temperaturniveau auf ein zweites Temperaturniveau unter Gewinnung genau einer ersten Flüssigfraktion und genau einer ersten Gasfraktion einmalig teilzukondensieren, zumindest einen Teil der ersten Gasfraktion auf dem ersten Druckniveau durch weiteres Abkühlen auf ein drittes Temperaturniveau unter Erhalt einer zweiten Flüssigfraktion und genau einer zweiten Gasfraktion einmalig teilzukondensieren, zumindest einen Teil der zweiten Gasfraktion auf dem ersten Druckniveau unter Erhalt genau einer dritten Flüssigfraktion und genau einer dritten Gasfraktion einer Gegenstromabsorption im Gegenstrom zu einer überwiegend Methan enthaltenden Absorptionsflüssigkeit zu unterwerfen, die erste, die zweite und die dritte Flüssigfraktion zumindest teilweise zu vereinigen und zumindest teilweise auf einem zweiten Druckniveau oberhalb des ersten Druckniveaus unter Erhalt einer Sumpfflüssigkeit und eines Kopfgases einer Tieftemperaturrektifikation zu unterwerfen, zumindest einen Teil des Kopfgases auf dem zweiten Druckniveau durch weiteres Abkühlen von auf ein viertes Temperaturniveau unter Erhalt einer vierten Flüssigfraktion und einer vierten Gasfraktion einmalig teilzukondensieren, und die überwiegend Methan enthaltende Absorptionsflüssigkeit durch weiteres Abkühlen zumindest eines Teils der vierten Gasfraktion von dem dritten auf ein fünftes Temperaturniveau zu bilden.

Eine entsprechende Anlage ist insbesondere dazu eingerichtet, ein Verfahren auszuführen, wie es zuvor erläutert wurde. Auf die genannten Merkmale und Vorteile kann daher an dieser Stelle verwiesen werden.

Die Erfindung wird nachfolgend unter Bezugnahme auf die beigefügte Zeichnung näher erläutert, die eine Ausführungsform der Erfindung veranschaulicht.

### Kurze Beschreibung der Zeichnungen

Figur 1 veranschaulicht ein Verfahren gemäß einer Ausführungsform der Erfindung.

### Ausführliche Beschreibung der Zeichnungen

In Figur 1 ist ein Verfahren gemäß einer besonders bevorzugten Ausführungsform der Erfindung in Form eines schematischen Prozessflussdiagramms veranschaulicht und insgesamt mit 100 bezeichnet. Die das Verfahren 100 betreffenden Erläuterungen gelten zugleich für eine entsprechende Anlage, so dass, falls auf Verfahrensschritte Bezug genommen wird, die entsprechenden Erläuterungen zugleich Anlagenkomponenten betreffen und umgekehrt.

In dem Verfahren 100 wird ein überwiegend Methan, Wasserstoff und Kohlenwasserstoffe mit zwei Kohlenstoffatomen enthaltendes Gasgemisch, das zuvor beispielsweise einer Hydrierung zur Umsetzung zuvor enthaltenen Acetylens unterworfen werden kann, und das aus dem Spaltgas eines nicht dargestellten Dampfspaltverfahrens gebildet wird, als Trenneinsatz gasförmig bereitgestellt.

Der Trenneinsatz wird in Form eines Stoffstroms a in einem Wärmetauscher 1 gegen eine Wasserstofffraktion (Stoffstrom b), eine Methanfraktion (Stoffstrom c), Hochdruckethylen (Stoffstrom d) und Mitteldruckethylen (Stoffstrom e) auf einem Druckniveau von ca. 34,9 bar (hier als "erstes Druckniveau" bezeichnet) ausgehend von einem Temperaturniveau bei etwa -23 °C ("erstes Temperaturniveau") auf ein Temperaturniveau von ca. -78 °C ("zweites Temperaturniveau") abgekühlt, dabei einmalig teilweise kondensiert und anschließend auf einem Druckniveau von ca. 34,7 bar (also noch dem ersten Druckniveau) in den Sumpfbereich 21 einer Absorptionskolonne 2, des C2-Absorbers, geführt. Dort scheidet sich das anfallende Kondensat ("erste Flüssigfraktion") von der Gasphase f ("erste Gasfraktion") ab.

Die erste Flüssigfraktion ist an Kohlenwasserstoffen mit zwei Kohlenstoffatomen angereichert. Aufgrund der einstufigen Teilkondensation scheidet sich eine vergleichsweise große Menge an Methan aus dem Trenneinsatz in der ersten Flüssigfraktion ab. Diese ist größer als sie in einer herkömmlichen mehrstufigen Teilkondensation wäre, wie sie EP 3 029 017 A1 unter Bezugnahme auf die Figuren 1 und 2 veranschaulicht ist. Die erste Gasfraktion enthält im Wesentlichen sämtliche Komponenten des Trenneinsatzes, ist jedoch insbesondere an Kohlenwasserstoffen mit zwei Kohlenstoffatomen abgereichert. Im Vergleich zu herkömmlichen mehrstufigen Teilkondensationen erhält sie aus den erwähnten Gründen auch weniger Methan.

Die erste Gasfraktion wird in Form eines Stoffstroms f im dargestellten Beispiel vollständig aus dem Sumpfbereich 21 der Absorptionskolonne 2 abgezogen und in einem Wärmetauscher 3 weiter gegen die bereits erwähnte Wasserstoff- und Methanfraktion (Stoffströme b und c) sowie gegen Niederdruckethylen (Stoffstrom g), auf ein Temperaturniveau von ca. -103 °C ("drittes Temperaturniveau") abgekühlt und ihrerseits teilweise kondensiert. Aufgrund der Verwendung der Stoffströme b und c ist das dritte Temperaturniveau tiefer, als es mit Niederdruckethylen alleine erzielbar wäre. Danach wird dieser Stoffstrom f, noch als Zweiphasengemisch, oberhalb einer Flüssigkeitssperre 22, der den Sumpfbereich 21 der Absorptionskolonne 2 von einem darüber liegenden Absorptionsbereich 23 trennt, wieder in die Absorptionskolonne 2 zurückgeführt. Die Flüssigkeitssperre 22 ermöglicht ein Ablaufen sich in einem unteren Bereich des Absorptionsbereichs 23 sammelnder Flüssigkeit und verhindert ein Aufsteigen von Gas aus dem Sumpfbereich 21 in den Absorptionsbereich 23.

Um den Druckverlust in dem Wärmetauscher 3 zu überwinden, wird dieser geodätisch oberhalb der Absorptionskolonne 2 angeordnet. Die Absorptionskolonne 2 arbeitet auf einem Druckniveau von ca. 34 bis 35 bar, also ebenfalls auf dem bereits mehrfach erwähnten ersten Druckniveau.

In der Absorptionskolonne 2 bzw. deren Absorptionsbereich erfolgt eine Phasentrennung des Stoffstroms f bzw. der entsprechend gekühlten ersten Gasfraktion. Die flüssige Phase ("zweite Flüssigfraktion") sammelt sich oberhalb der Flüssigkeitssperre 22 und vereinigt sich dort mit von oben herabrieselnder beladener Absorptionsflüssigkeit ("dritte Flüssigfraktion"). Der bei der Phasentrennung des Stoffstroms f bzw. der entsprechend gekühlten ersten Gasfraktion gasförmig verbliebene Anteil ("zweite Flüssigfraktion") steigt in dem Absorptionsbereich auf und wird dabei einer Gegenstromabsorption im Gegenstrom zu einer überwiegend Methan enthaltenden Absorptionsflüssigkeit in Form eines Stoffstroms n unterworfen.

Bei der Gegenstromabsorption bildet sich eine flüssige Fraktion (die erwähnte "dritte Flüssigfraktion"), die sich, wie erwähnt, mit der zweiten Flüssigfraktion vereinigt. Die zweite und dritte Flüssigfraktion fließen vereinigt über die Flüssigkeitssperre 22 in den Sumpfbereich 21 der Absorptionskolonne 2 ab, wo sie sich mit der ersten Flüssigfraktion vereinigen. Die bei der Gegenstromabsorption gasförmig verbleibende Fraktion ("dritte Gasfraktion") steigt auf und wird in Form eines Stoffstroms o aus der Absorptionskolonne 2 abgezogen.

Aus dem Sumpfbereich 21 der Absorptionskolonne 2, werden die vereinigte erste, zweite und dritte Flüssigfraktion mittels einer Sumpfpumpe 4 bei einer Temperatur von ca. -79 °C (also noch auf dem ersten Temperaturnive au) aus der Absorptionskolonne 2, genauer dem Sumpfbereich 21, abgezogen, und in eine Rektifikationskolonne 5, den sogenannten Demethanizer, gepumpt (Stoffstrom h). Durch die Wirkung der Sumpfpumpe 4 erfolgt eine Druckerhöhung auf ca. 38 bar. In der Rektifikationskolonne 5 werden die Kohlenwasserstoffe mit zwei Kohlenstoffatomen, also das mehrfach erwähnte "Trennprodukt", auf einem Druckniveau von ca. 35 bar ("zweites Druckniveau") von Methan und leichteren Komponenten getrennt und verlassen die Rektifikationskolonne 5 über den Sumpf als Sumpfflüssigkeit in Form eines Stoffstroms i. Die Rektifikationskolonne 5 arbeitet allgemein auf dem zweiten Druckniveau, insbesondere bei ca. 35 bis 36 bar, ihr Sumpf wird in einem Sumpfverdampfer 52 mit Hochdruckpropylen aufgekocht. Der Stoffstrom i, also das Trennprodukt, kann in dem Wärmetauscher 1 angewärmt und einem weiteren Trennschritt zur Trennung der Kohlenwasserstoffe mit zwei Kohlenstoffatomen voneinander zugeführt werden.

Kopfgas der Rektifikationskolonne 5 wird in Form eines Stoffstroms k in einem Wärmetauscher 6 unter Verwendung von Niederdruckethylen, das auf einem Temperaturniveau von ca. -101 °C vorliegt, bis auf ein Temperaturniveau von ca. - 98 °C ("viertes Temperaturniveau") abgekühlt und teilweise kondensiert. Der Wärmetauscher 6 ist im Kopf der Rektifikationskolonne 5 eingebaut, so dass das anfallende Kondensat ("vierte Flüssigfraktion") ohne Pumpe nur durch die Schwerkraft als Rücklauf in Form eines Stoffstroms I zurück in die Rektifikationskolonne 5 fließt. Weil hier nur Niederdruckethylen eingesetzt wird, liegt das vierte Temperaturniveau oberhalb des dritten Temperaturniveaus, das durch den Wärmetauscher 3 bereitgestellt wird. Das verbleibende Gas, ("vierte Gasfraktion") besteht vorwiegend aus Methan und verlässt die Rektifikationskolonne 5 über Kopf in Form eines Stoffstroms m. Der Großteil dieses Stoffstroms m wird in Form eines Stoffstroms n in einem Wärmetauscher 7 weiter auf ein Temperaturniveau ca. -152 °C ("fünftes Temperaturniveau") abgekühlt, im Zuge dessen zum überwiegenden Teil kondensiert, und anschließend, wie bereits erwähnt, als Rücklauf dem Absorptionsbereich 23 der Absorptionskolonne 2 zugeführt.

Das Kopfprodukt des Absorptionsbereichs 23 der Absorptionskolonne 2 (also die dritte Gasfraktion) wird in Form eines Stoffstroms o, der auf einem Druckniveau von ca. 34,4 bar vorliegt (also dem ersten Druckniveau), in dem Wärmetauscher 7 ebenfalls auf das fünfte Temperaturniveau von ca. -152 °C abgekühlt und teilweise kondensiert. In einem Abscheidebehälter 8 wird das anfallende Kondensat, die sogenannte Methanfraktion ("fünfte Flüssigfraktion"), von der Gasphase, der sogenannten Wasserstofffraktion ("fünfte Gasfraktion"), abgetrennt. Die Methanfraktion, hier zunächst noch mit p bezeichnet, wird erst auf ein geeignetes Druckniveau, beispielsweise eines Heizgasnetzes, entspannt und anschließend in den Wärmetauschern 7, 3 und 1 angewärmt.

Zur Kältebilanzierung des Wärmetauschers 7 wird der Rektifikationskolonne 5 oberhalb eines Flüssigkeitsbodens 51 flüssiges Methan entnommen und in Form eines Stoffstroms q der Methanfraktion des Stoffstroms p zugeführt, nachdem es in im Wärmetauscher 7 auf das fünfte Temperaturniveau von ca. -152 °C abgekühlt wurde. Ebenfalls kann ein kleiner Teil des Stoffstroms m in Form eines Stoffstroms r dem Stoffstrom p zugespeist werden. Der aus den Stoffströmen p, q und r gebildete Sammelstrom wird noch immer als Methanfraktion bezeichnet und ist in Form des bereits erwähnten Stoffstroms c veranschaulicht.

Die Gasphase mit ca. 90 mol-% Wasserstoff aus dem Abscheidebehälter 8 wird, wie die Methanfraktion der Stoffströme p bzw. c, in den Wärmetauschern 7, 3 und 1 gegen den warmen Stoffstrom a angewärmt.

## Patentansprüche

1. Verfahren (100) zur Gewinnung eines Trennprodukts, das überwiegend Kohlenwasserstoffe mit zwei Kohlenstoffatomen enthält, unter Verwendung eines Trenneinsatzes, der überwiegend Methan, Wasserstoff und Kohlenwasserstoffe mit zwei Kohlenstoffatomen enthält, wobei der Methangehalt des Trenneinsatzes bis zu 30% auf molarer Basis beträgt und der Trenneinsatz in gasförmigem Zustand bereitgestellt wird, **dadurch gekennzeichnet, dass**
- der Trenneinsatz auf einem ersten Druckniveau durch Abkühlen von einem ersten Temperaturniveau von -20 bis -35 °C auf ein zweites Temperaturniveau von -75 bis -80 °C unter Gewinnung genau einer ersten Flüssigfraktion und genau einer ersten Gasfraktion einmalig teilkondensiert wird,
- zumindest ein Teil der ersten Gasfraktion auf dem ersten Druckniveau durch weiteres Abkühlen auf ein drittes Temperaturniveau unter Erhalt genau einer zweiten Flüssigfraktion und genau einer zweiten Gasfraktion einmalig teilkondensiert wird,
- zumindest ein Teil der zweiten Gasfraktion auf dem ersten Druckniveau unter Erhalt genau einer dritten Flüssigfraktion und genau einer dritten Gasfraktion einer Gegenstromabsorption im Gegenstrom zu einer überwiegend Methan enthaltenden Absorptionsflüssigkeit unterworfen wird,
- die erste, die zweite und die dritte Flüssigfraktion zumindest teilweise vereinigt und zumindest teilweise auf einem zweiten Druckniveau oberhalb des ersten Druckniveaus unter Erhalt einer Sumpfflüssigkeit und eines Kopfgases einer Tieftemperaturrektifikation unterworfen werden,
- zumindest ein Teil des Kopfgases auf dem zweiten Druckniveau durch weiteres Abkühlen auf ein viertes Temperaturniveau unter Erhalt einer vierten Flüssigfraktion und einer vierten Gasfraktion einmalig teilkondensiert wird, und
- die überwiegend Methan enthaltende Absorptionsflüssigkeit durch weiteres Abkühlen zumindest eines Teils der vierten Gasfraktion auf ein fünftes Temperaturniveau gebildet wird.

2. Verfahren nach Anspruch 1, bei dem das dritte Temperaturniveau bei -100 bis -105 °C und/oder das vierte Temperaturniveau bei -9 5 bis -100 °C und/oder das fünfte Temperaturniveau bei -140 bis -155 °C liegt.

3. Verfahren nach Anspruch 1 oder 2, bei dem das erste Druckniveau bei 32 bis 37 bar und/oder das zweite Druckniveau bei 35 bis 40 bar liegt.

4. Verfahren nach einem der vorstehenden Ansprüche, bei dem für die Gegenstromabsorption eine Absorptionskolonne (2) verwendet wird, die einen Sumpfbereich (21) und einen von dem Sumpfbereich (21) durch eine Flüssigkeitssperre (22) getrennten Absorptionsbereich (23), der oberhalb des Sumpfbereichs (21) angeordnet ist, aufweist, wobei die Flüssigkeitssperre (22) derart ausgebildet ist, dass sie Flüssigkeit, die sich in einem unteren Bereich des Absorptionsbereichs (23) an der Flüssigkeitssperre (22) sammelt, in den Sumpfbereich (21) abfließen lässt und dabei ein Aufsteigen von Gas aus dem Sumpfbereich (21) in den Absorptionsbereich (23) verhindert.

5. Verfahren nach Anspruch 4, bei dem der von dem ersten Temperaturniveau auf das zweite Temperaturniveau abgekühlte Trenneinsatz als Zweiphasengemisch in den Sumpfbereich (21) eingespeist wird, wobei sich in diesem die erste Flüssigfraktion von der ersten Gasfraktion trennt.

6. Verfahren nach Anspruch 5, bei dem die auf das dritte Temperaturniveau weiter abgekühlte erste Gasfraktion oder deren Teil als Zweiphasengemisch sumpfseitig in den Absorptionsbereich (23) eingespeist wird, wobei sich in diesem die zweite Flüssigfraktion von der zweiten Gasfraktion trennt.

7. Verfahren nach Anspruch 6, bei dem die dritte Flüssigfraktion mit der zweiten Flüssigfraktion bei deren Bildung oberhalb der Flüssigkeitssperre (22) vereinigt und über die Flüssigkeitssperre (22) in den Sumpfbereich abgelassen werden, wo sie bei deren Bildung mit der ersten Flüssigfraktion vereinigt werden.

8. Verfahren nach einem der vorstehenden Ansprüche, bei dem die erste, die zweite und die dritte Flüssigfraktion oder deren vereinigte Anteile mittels einer Sumpfpumpe (4) druckerhöht und in eine für die Tieftemperaturrektifikation verwendete Rektifikationskolonne (5) überführt werden.

9. Verfahren nach Anspruch 8, bei dem zur Teilkondensation des Kopfgases oder dessen Teils ein Kopfkondensator (6) der Rektifikationskolonne (5) verwendet wird, der unter Verwendung von Niederdruckethylen gekühlt wird.

10. Verfahren nach einem der vorstehenden Ansprüche, bei dem zumindest ein Teil der dritten Gasfraktion auf dem ersten Druckniveau durch Abkühlen auf das fünfte Temperaturniveau unter Erhalt genau einer fünften Flüssigfraktion und genau einer fünften Gasfraktion einmalig teilkondensiert wird.

11. Verfahren nach Anspruch 10, bei dem zur Abkühlung der dritten und der vierten Gasfraktion oder deren Teile zumindest ein Wärmetauscher (7) verwendet wird, der unter Verwendung zumindest eines Teils der fünften Flüssigfraktion und der fünften Gasfraktion gekühlt wird.

12. Verfahren nach Anspruch 10 oder 11, bei dem zur Abkühlung des Trenneinsatzes zumindest ein Wärmetauscher (1) verwendet wird, der unter Verwendung zumindest eines Teils der fünften Flüssigfraktion und der fünften Gasfraktion sowie mit Hoch- und Mitteldruckethylen gekühlt wird.

13. Verfahren nach einem der vorstehenden Ansprüche, bei dem zur Abkühlung der ersten Gasfraktion wenigstens ein Wärmetauscher (3) verwendet wird, der unter Verwendung zumindest eines Teils der fünften Flüssigfraktion und der fünften Gasfraktion sowie mit Niederdruckethylen gekühlt wird.

## Claims

1. Method (100) for obtaining a separation product which contains mostly hydrocarbons having two carbon atoms, using a separation feedstock which contains mostly methane, hydrogen and hydrocarbons having two carbon atoms, the methane content of the separation feedstock being up to 30 % on a molar basis and the separation feedstock being provided in gaseous state, **characterized in that**
- the separation feedstock is partially condensed once at a first pressure level by cooling from a first temperature level of from -20 to -35 °C to a second temperature level of from -75 to -80 °C, obtaining precisely one first liquid fraction and precisely one first gas fraction,
- at least a portion of the first gas fraction at the first pressure level is partially condensed once by further cooling to a third temperature level, obtaining precisely one second liquid fraction and precisely one second gas fraction,
- at least a portion of the second gas fraction at the first pressure level is subjected to counter-current absorption in counter-current to a mostly methane-containing absorption liquid, obtaining precisely one third liquid fraction and precisely one third gas fraction,
- the first, the second and the third liquid fractions are at least partially combined and at least partially subjected to cryogenic rectification at a second pressure level above the first pressure level, obtaining a sump liquid and a top gas,
- at least a portion of the top gas at the second pressure level is partially condensed once by further cooling to a fourth temperature level, obtaining a fourth liquid fraction and a fourth gas fraction, and
- the absorption liquid containing mostly methane is formed by further cooling of at least a portion of the fourth gas fraction to a fifth temperature level.

2. Method according to claim 1, wherein the third temperature level is at -100 to -105 °C and/or the fourth temperature level is at -95 to -100 °C and/or the fifth temperature level is at -140 to -155 °C.

3. Method according to claim 1 or 2, wherein the first pressure level is at 32 to 37 bar and/or the second pressure level is at 35 to 40 bar.

4. Method according to any preceding claim, wherein an absorption column (2) is used for the counter-current absorption, having a sump region (21) and an absorption region (23) that is separated from the sump region (21) by a liquid barrier (22) and is arranged above the sump region (21), wherein the liquid barrier (22) is configured such that it drains any liquid accumulating in a lower area of the absorption region (23) at the liquid barrier (22) into the sump region (21) and thereby prevents gas from rising from the sump region (21) into the absorption region (23).

5. Method according to claim 4, wherein the separation feedstock cooled from the first temperature level to the second temperature level is fed into the sump region (21) as a two-phase mixture, wherein the first liquid fraction separates from the first gas fraction therein.

6. Method according to claim 5, wherein the first gas fraction further cooled to the third temperature level or the portion thereof is fed into the absorption region (23) as a two-phase mixture at the sump, wherein the second liquid fraction separates from the second gas fraction therein.

7. Method according to claim 6, wherein the third liquid fraction is combined with the second liquid fraction when it is formed above the liquid barrier (22) and discharged via the liquid barrier (22) into the sump region, where they are combined with the first liquid fraction when it is formed.

8. Method according to any preceding claim, wherein the first, the second, and the third liquid fraction or the combined portions thereof are pressure-increased by means of a sump pump (4) and transferred to a rectification column (5) used for cryogenic rectification.

9. Method according to claim 8, wherein a top condenser (6) of the rectification column (5) is used for partial condensation of the top gas or the portion thereof which is cooled using low-pressure ethylene.

10. Method according to any preceding claim, wherein at least a portion of the third gas fraction at the first pressure level is partially condensed one time by cooling to the fifth temperature level, obtaining precisely one fifth liquid fraction and precisely one fifth gas fraction.

11. Method according to claim 10, wherein at least one heat exchanger (7) is used to cool the third and the fourth gas fraction or the portions thereof, which is cooled using at least a portion of the fifth liquid fraction and the fifth gas fraction.

12. Method according to claim 10 or 11, wherein at least one heat exchanger (1) is used to cool the separation feedstock, which is cooled using at least a portion of the fifth liquid fraction and of the fifth gas fraction as well as with highpressure and medium-pressure ethylene.

13. Method according to any preceding claim, wherein at least one heat exchanger (3) is used to cool the first gas fraction, which is cooled using at least a portion of the fifth liquid fraction and of the fifth gas fraction as well as with low-pressure ethylene.

## Revendications

1. Procédé (100) de production d'un produit de séparation, lequel contient principalement des hydrocarbures à deux atomes de carbone, à l'aide d'un insert de séparation, lequel contient principalement du méthane, de l'hydrogène et des hydrocarbures à deux atomes de carbone, la teneur en méthane de l'insert de séparation représentant jusqu'à 30 % sur une base molaire, et l'insert de séparation étant mis à disposition à l'état gazeux, **caractérisé en ce que**
- l'insert de séparation à un premier niveau de pression est partiellement condensé, une seule fois, par refroidissement à partir d'un premier niveau de température de -20 à -35 °C à un deuxième niveau de température de -75 à - 80 °C, avec production d'exactement une première fraction liquide et d'exactement une première fraction gazeuse,
- au moins une partie de la première fraction gazeuse au premier niveau de pression est partiellement condensée, une seule fois, par refroidissement supplémentaire à un troisième niveau de température avec obtention d'exactement une deuxième fraction liquide et d'exactement une deuxième fraction gazeuse,
- au moins une partie de la deuxième fraction gazeuse au premier niveau de pression est soumise à une absorption à contre-courant, à contre-courant par rapport à un liquide d'absorption contenant principalement du méthane, avec obtention d'exactement une troisième fraction liquide et d'exactement une troisième fraction gazeuse,
- les première, deuxième et troisième fractions liquides sont au moins partiellement réunies et au moins partiellement soumises, à un deuxième niveau de pression supérieur au premier niveau de pression, à une rectification à basse température avec obtention d'un liquide de fond et d'un gaz de tête,
- au moins une partie du gaz de tête au deuxième niveau de pression est partiellement condensée, une seule fois, par refroidissement supplémentaire à un quatrième niveau de température avec obtention d'une quatrième fraction liquide et d'une quatrième fraction gazeuse et
- le liquide d'absorption contenant principalement du méthane est formé par refroidissement supplémentaire d'au moins une partie de la quatrième fraction gazeuse à un cinquième niveau de température.

2. Procédé selon la revendication 1, dans lequel le troisième niveau de température est situé à -100 jusqu'à -105 °C et/ou le quatrième niveau de température est situé à -95 jusqu'à -100 °C et/ou le cinquième niveau de température est situé à -140 jusqu'à -155 °C.

3. Procédé selon la revendication 1 ou 2, dans lequel le premier niveau de pression est situé à 32 jusqu'à 37 bars et/ou le deuxième niveau de pression est situé à 35 jusqu'à 40 bars.

4. Procédé selon l'une quelconque des revendications précédentes, dans lequel, pour l'absorption à contre-courant, une colonne d'absorption (2) est utilisée, laquelle comprend une zone de fond (21) et une zone d'absorption (23) séparée de la zone de fond (21) par une barrière liquide (22), laquelle est disposée au-dessus de la zone de fond (21), la barrière liquide (22) étant conçue de telle sorte qu'elle laisse s'écouler le liquide, lequel s'accumule dans une zone inférieure de la zone d'absorption (23) au niveau de la barrière liquide (22), dans la zone de fond (21) et empêche ainsi le gaz de monter à partir de la zone de fond (21) dans la zone d'absorption (23).

5. Procédé selon la revendication 4, dans lequel l'insert de séparation refroidi à partir du premier niveau de température au deuxième niveau de température est injecté sous la forme de mélange à deux phases dans la zone de fond (21), la première fraction liquide se séparant dans celle-ci de la première fraction gazeuse.

6. Procédé selon la revendication 5, dans lequel la première fraction gazeuse refroidie de manière supplémentaire au troisième niveau de température ou sa partie est injectée sous la forme d'un mélange à deux phases à partir du fond dans la zone d'absorption (23), la deuxième fraction liquide se séparant dans celle-ci de la deuxième fraction gazeuse.

7. Procédé selon la revendication 6, dans lequel la troisième fraction liquide et la deuxième fraction liquide sont réunies lors de leur formation au-dessus de la barrière liquide (22) et évacuées via la barrière liquide (22) dans la zone de fond, où elles sont réunies avec la première fraction liquide lors de sa formation.

8. Procédé selon l'une quelconque des revendications précédentes, dans lequel la pression des première, deuxième et troisième fractions liquides ou leurs proportions réunies est augmentée au moyen d'une pompe de fond (4) et elles sont transférées dans une colonne de rectification (5) utilisée pour la rectification à basse température.

9. Procédé selon la revendication 8, dans lequel, pour la condensation partielle du gaz de tête ou de la partie de celui-ci, un condenseur de tête (6) de la colonne de rectification (5) est utilisé, lequel est refroidi à l'aide d'éthylène basse pression.

10. Procédé selon l'une quelconque des revendications précédentes, dans lequel au moins une partie de la troisième fraction gazeuse au premier niveau de pression est partiellement condensée, une seule fois, par refroidissement au cinquième niveau de température avec obtention d'exactement une cinquième fraction liquide et d'exactement une cinquième fraction gazeuse.

11. Procédé selon la revendication 10, dans lequel, pour le refroidissement des troisième et quatrième fractions gazeuses ou de leurs parties, au moins un échangeur de chaleur (7) est utilisé, lequel est refroidi à l'aide d'au moins une partie de la cinquième fraction liquide et de la cinquième fraction gazeuse.

12. Procédé selon la revendication 10 ou 11, dans lequel, pour le refroidissement de l'insert de séparation, au moins un échangeur de chaleur (1) est utilisé, lequel est refroidi à l'aide d'au moins une partie de la cinquième fraction liquide et de la cinquième fraction gazeuse, ainsi qu'à l'aide d'éthylène haute et moyenne pression.

13. Procédé selon l'une quelconque des revendications précédentes, dans lequel, pour refroidir la première fraction gazeuse, au moins un échangeur de chaleur (3) est utilisé, lequel est refroidi à l'aide d'au moins une partie de la cinquième fraction liquide et de la cinquième fraction gazeuse, ainsi qu'à l'aide d'éthylène basse pression.
